(19) Europäisches Patentamt
European Patent Office
Office européen des brevets

(11) Veröffentlichungsnummer: **0 263 463 B1**

# (12) EUROPÄISCHE PATENTSCHRIFT

(45) Veröffentlichungstag der Patentschrift: **25.03.92**

(51) Int. Cl.5: **C07D 487/08**, //(C07D487/08, 241:00,241:00)

(21) Anmeldenummer: **87114504.1**

(22) Anmeldetag: **05.10.87**

## (54) Verfahren zur Herstellung von 1,4-Diazabicyclo(2,2,2)-octanen.

(30) Priorität: **08.10.86 DE 3634260**
**08.10.86 DE 3634257**
**08.10.86 DE 3634258**

(43) Veröffentlichungstag der Anmeldung:
**13.04.88 Patentblatt 88/15**

(45) Bekanntmachung des Hinweises auf die Patenterteilung:
**25.03.92 Patentblatt 92/13**

(84) Benannte Vertragsstaaten:
**BE DE FR GB NL**

(56) Entgegenhaltungen:
**EP-A- 0 111 928**
**EP-A- 0 158 319**
**EP-A- 0 158 975**
**DE-A- 2 434 913**
**US-A- 3 285 920**

(73) Patentinhaber: **BASF Aktiengesellschaft**
**Carl-Bosch-Strasse 38**
**W-6700 Ludwigshafen(DE)**

(72) Erfinder: **Hölderich, Wolfgang, Dr.**
**Mannheimer Strasse 18c**
**W-6710 Frankenthal(DE)**
Erfinder: **Schneider, Kurt, Dr.**
**Auf dem Koeppel**
**W-6702 Bad Duerkheim(DE)**
Erfinder: **Lermer, Helmut, Dr.**
**D 3,4**
**W-6800 Mannheim 1(DE)**
Erfinder: **Best, Walter, Dr.**
**Im Spiess 2**
**W-6714 Weisenheim(DE)**

EP 0 263 463 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 1,4-Diazabicyclo(2,2,2)-octain (DABCO) und dessen C-substituierte Derivate durch Umsetzung heterocyclischer Amine in Gegenwart von L-Zeolithen, Phosphaten mit Zeolithstruktur und Zirkonphosphaten als Katalysatoren.

Es ist bekannt, daß man DABCO und seine Derivate aus heterocyclischen Aminen auf katalytischem Wege in Gegenwart von Phosphaten des Ca, Sr, Ba, Zn, La, Al, Co, Ni oder Ce als Heterogenkatalysatoren (EP 111 928, US 4 514 567, US 3 342 820, US 3 297 701 und EP 60 322) herstellen kann. Derartig gefällte Phosphate liefern unbefriedigende Ausbeuten.

Es ist auch bekannt, daß man Aluminiumsilikatzeolithe des Pentasiltyps als Heterogenkatalysatoren für obige Reaktion einsetzen kann (EP 158 319). Die Ausbeuten sind klein; bei niedrigem Umsatz werden zwar hohe Selektivitäten, bei hohem Umsatz jedoch niedrige Selektivitäten gefunden. Bei Einsatz von A-Zeolith (DE-PS 24 34 913) und von Silica-Alumina-Crackkatalysatoren (US 3 166 558) werden ebenfalls nur niedrige Ausbeuten erzielt. Weiterhin weist das Reaktionsprodukt eine Reihe unerwünschter Nebenprodukte wie N-Alkylpiperazine und Pyrazin-Derivate auf. Die Nebenprodukte sind in ihren physikalischen und chemischen Eigenschaften dem DABCO ähnlich und daher mit den üblichen Methoden, wie Destillation und Kristallisation, nur unvollständig abzutrennen. Für die Weiterverarbeitung werden an die Reinheit des DABCO hohe Anforderungen gestellt und erfordern daher eine aufwendige und kostspielige Reinigung.

Es wurde gefunden, daß man 1,4-Diazabicyclo(2,2,2)-octan und C-substituierte 1,4-Diazabicyclo(2,2,2)-octane der Formel (I)

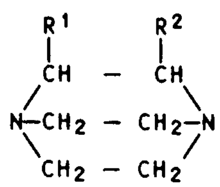

(I),

in der $R^1$ und $R^2$ Wasserstoff-, Alkyl- mit 1 bis 4 C-Atomen oder Alkenylreste mit bis zu 4 C-Atomen bedeuten können, in hohen Ausbeuten (Umsatz x Selektivität) erhält, wenn man heterocyclische Amine der Formel (II)

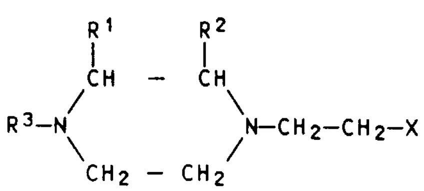

(II),

in der $R^1$ und $R^2$ obige Bedeutung haben und $R^3$ Wasserstoff-, Hydroxyethyl-, Aminoethyl- und X Hydroxyl-, Amino-, Hydroxyethyl- oder Aminoethylreste bedeuten, in Gegenwart von L-Zeolithen, Phosphaten mit Zeolithstruktur und Zirkonphosphaten als Katalysatoren umsetzt.

Als Ausgangsstoffe sind Verbindungen der Formel II wie z.B. Monohydroxyethylpiperazin, Dihydroxyethylpiperazin, Monoaminoethylpiperazin oder Aminoethylhydroxyethylpiperazin geeignet.

Als Katalysatoren für das erfindungsgemäßen Verfahren kann man u.a. L-Zeolithe verwenden. Zeolithe sind kristalline Aluminiumsilikate, die eine hochgeordnete Struktur mit einem starren dreidimensionalen Netzwerk von $SiO_4$- und $Al0_4$-Tetraedern besitzen, die durch gemeinsame Sauerstoffatome verbunden sind. Das Verhältnis der Si- und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Der L-Zeolith kann nach seiner Isolierung, Trocknung bei 100 bis 160°C, vorzugsweise 110°C und Calcinierung bei 450 bis 550°C, vorzugsweise 500 bis 540°C, mit einem Bindemittel im Verhältnis 90 : 10

bis 40 : 60 Gew.-% zu Strängen oder Tabeletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte L-Zeolith direkt nach der Trocknung verformt wird und erst nach der Verformung einer Calcinierung unterworfen wird. Die hergestellten Zeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten L-Zeolithen in einem Steigrohr vorlegt und bei 20 bis 100°C eine wäßrige oder ammonialkalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wir der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80°C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400°C bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2

n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160 °C, getrocknet und bei Temperaturen von im allgemeinen 450 °C bis 600 °C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5 h, vorzugsweise mit 12 bis 20 Gew.-%iger Salzsäure, behandelt. Zweckmäßig wird das zeolithische Material anschließend ausgewaschen, bei Temperaturen von 100 bis 160 °C getrocknet und bei Temperaturen von 450 bis 600 °C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Als Katalysatoren für das erfindungsgemäßen Verfahren werden Phosphate mit Zeolithstruktur eingesetzt. Diese Phosphate werden vorzugsweise unter hydrothermalen Bedingungen synthetisiert.

Als Katalysatoren kann man hydrothermal hergestellte Aluminiumphosphate, Siliciumaluminiumphosphte, Siliciumeisenaluminiumphosphate, Boraluminiumphosphate oder Eisenaluminiumphosphate verwenden.

Als Aluminiumphosphat-Katalysatoren werden für das erfindungsgemäße Verfahren, insbesondere unter hydrothermalen Bedingungen, synthetisierte Aluminiumphosphate eingesetzt. Geeignete Aluminiumphosphate sind z.B. APO-5, APO-9, APO-11, APO-12, APO-14, APO-21, APO-25, APO-31 und APO-33.

Beispielsweise $AlPO_4$-5 (APO-5) wird synthetisiert, indem man Orthophosphorsäure mit Pseudoboehmit (Catapal SB ®) in Wasser homogen mischt; zu dieser Mischung Tetrapropylammoniumhydroxid gibt und danach bei etwa 150 °C 20 bis 60 h unter autogenem Druck in enem Autoklaven umsetzt. Das abfiltrierte $AlPO_4$ wird getrocknet bei 100 bis 160 °C und calciniert bei 450 bis 550 °C.

$AlPO_4$-9 (APO-9) wird ebenfalls aus Orthophosphorsäure und Pseudoboehmit aber in wäßriger DABCO-Lösung (1,4-Diazabicyclo-(2,2,2)octan) bei ca. 200 °C unter autogenem Druck während 200 bis 400 h synthetisiert. Verwendet man anstelle DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidon-Lösung bei 150 bis 200 °C unter autogenem Druck während 50 bis 200 h.

Für das erfindungsgemäße Verfahren kann man auch bekannte Siliciumaluminiumphosphate wie SAPO-5, SAPO-11, SAPO-31 und SAPO-34 einsetzen. Diese Verbindungen werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C, während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Siliciumaluminiumphosphate sind auch ZYT-5, ZYT-6, ZYT-7, ZYT-9, ZYTI-11 und ZYT-12 geeignet.

Als Siliciumaluminumphosphate sind weiter die MCM-Typen, z.B. MCM-1 bis MCM-10 geeignet. Auch weitere isomorphe Substitution mit Ge, Ti, Fe, Mn, Zn, Co, Mg, As, Be, B, Cr, Ga, Li, V und Zr führt zu mikroporösen, kristallinen, für das erfindungsgemäße Verfahren geeignete Katalysatoren.

Die so hergestellten Phosphate mit Zeolithstruktur können nach ihrer Isolierung, Trocknung bei 100 bis 160 °C, vorzugsweise 110 °C und Calcinierung bei 450 bis 550 °C, vorzugsweise 500 bis 540 °C, mit einem Bindemittel im Verhältnis 90 : 10 bis 40 : 60 Gew.% zu Strängen oder Tabletten verformt werden. Als bindemittel eignen sich diverse Aluminiumoxide, bevorzugt Boehmit, amorphe Aluminiumsilikate mit einem $SiO_2/Al_2O_3$-Verhältnis von 25 : 75 bis 25 : 5, Siliciumoxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110 °C/116 h getrocknet und bei 500 °C/16 h calciniert.

Man kann auch die isolierten Phosphate direkt nach der Trocknung verformen und erstmals nach der Verformung einer Calcinierung unterwerfen. Die Phosphate können auch in reiner Form, d.h. ohne Bindemittel, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Um eine möglichst große Selektivität, hohen Umsatz sowie lange Standzeiten zu erreichen, ist es mitunter zweckmäßig, die Phosphate zu modifizieren. Eine geeignete Modifizierung der Katalysatoren besteht z.B. darin, daß man die unverformten oder verformten Phosphate mit Metallsalzen durch Imprägnieren dotiert. Als Metalle für die Dotierung kann man Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppen wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn und Seltene Erdmetalle wie La, Ce, Pr, Nd, Er, Yb und U einsetzen.

Eine Möglichkeit der Metallaufbringung auf das Phosphat besteht darin, daß man das Material mit einem Halogenid, Nitrat oder Oxid des Metalles in wäßriger, alkoholischer oder ammoniakalischer Lösung imprägniert. An eine Imprägnierung schließt sich zimindest eine Trocknung, wahlweise eine abermahlige Calcinierung an.

Eine mögliche Ausführungsform besteht z.B. darin, daß man $Cs_2CO_3$, $La(NO_3)_3$, $Cu(NO_3)_2$, $Ni(NO_3)_2$, $Ce(NO_3)_3$ oder $Co(NO_3)_2$ in Wasser löst. Mit dieser Lösung wird das verformte oder unverformte Phosphat eine gewisse Zeit, etwa 30 Minuten, getränkt. Die eventuell überstehende Lösung wird am Rotationsverdampfer von Wasser befreit. Danach wird das getränkte Phosphat bei etwa 150 ° C getrocknet und bei etwa 550 ° C calciniert. Der Tränkvorgang kann mehrmals hintereinander vorgenommen werden, um den gewünschten Metallgehalt einzustellen.

Es ist auch möglich, eine wäßrige $Cr(NO_3)_3$ oder eine ammoniakalische $Pd(NO_3)_2$-Lösung herzustellen und darin das reine pulverförmige Phosphat bei 40 bis 100 ° C unter Rühren etwa 24 h aufzuschlämmen. Nach Abfiltrieren, Trocknen bei etwa 150 ° C und Calcinierung bei etwa 500 ° C kann das gewonnene dotierte Phosphat mit oder ohne Bindemittel zu Strängen, Pellets oder Wirbelgut weiterverarbeitet werden.

Eine weitere Möglichkeit der Modifizierung besteht darin, daß man das Phosphat - verformt oder unverformt - einer Behandlung mit Säuren wie Salzsäure, Flußsäure und Phosphorsäure und/oder Wasserdampf unterwirft.

Weiterhin lassen sich für das erfindungsgemäße Verfahren Zirkonphosphate einsetzen.

Die Zirkonphosphate - hierzu gehören auch die Zirkonphosphorsilikate - können nach ihrer Isolierung, Trocknung bei 100 bis 160 ° C, vorzugsweise 110 ° C und Calcinierung bei 450 bis 550 ° C, vorzugsweise 500 bis 540 ° C, mit einem Bindemittel im Verhältnis 90 : 10 bis 40 : 60 Gew.% zu Strängen oder Tabletten verformt werden. Als Bindemittel eignen sich diverse Aluminiumoxide, bevorzugt Boehmit, amorphe Aluminiumsilikate mit einem $SiO_2/Al_2O_3$-Verhältnis von 25 : 75 bis 95 : 5, insbesondere 75 : 25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110 ° C/16 h getrocknet und bei 500 ° C/16 h calciniert.

Man erhält gut geeignete Katalysatoren, wenn die Zirkonphosphate und Zirkonphosphorsilikate direkt nach der Trocknung verformt werden und erstmals nach der Verformung einer Calcinierung unterworfen werden. Die Zirkonsphosphate und Zirkonphosphorsilikate können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden können.

Wenn bei der erfindungsgemäßen Verwendung der Katalysatoren eventuell eine durch Koksabscheidung bedingte Desaktivierung eintritt, empfiehlt es sich, die Katalysatoren durch Abbrennen der Koksablagerung mit Luft oder mit einem Luft/$N_2$-Gemisch bei 400 bis 550 ° C, bevorzugt 500 ° C, zu regenerieren. Die Katalysatoren erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4 mm-Stränge oder als Tabletten mit 3 bis 5 mm Durchmesser oder als Pulver mit Teilchengrößen von 0,1 bis 0,5 mm oder als Wirbelkontakt eingesetzt werden.

Das Verfahren wird vorzugsweise in der Gasphase bei 200 bis 550 ° C, insbesondere 300 bis 450 ° C, und einer Belastung WHSV = 0,1 bis 20 $h^{-1}$, insbesondere 0,5 bis 5 $h^{-1}$ (g Ausgangsstoffe/g Kataysator und Stunde) ausgeführt. Die Reaktion kann im Festbett oder im Wirbelbett ausgeführt werden. Im allgemeinen steigt der Umsatz mit steigender Temperatur an, während die Selektivität in einem bestimmten Temperaturbereich nur wenig zurückgeht. Es ist auch möglich, die Reaktion in der Flüssigphase (Suspension-, Riesel- oder Sumpffahrweise) durchzuführen. Das Verfahren kann bei Normaldruck oder erhöhtem Druck, diskontinuierlich oder vorzugsweise kontinuierlich durchgeführt werden.

Schwerflüchtige oder feste Ausgangsstoffe werden in gelöster Form, z.B. in $H_2O$-, THF-, Toluol- oder Petrolether-Lösung eingesetzt. Allgemein ist auch eine Verdünnung mit den genannten Lösungsmitteln oder

Inertgasen wie $N_2$, Ar, $H_2O$-Dampf möglich.

Nach der Umsetzung werden die entstandenen 1,4-Diazabicyclo(2,2,2)-octane nach üblichen Verfahren, z.B. durch Destillation aus dem Reaktionsgemisch, isoliert; nicht umgesetzte Ausgangsstoffe werden gegebenenfalls zurückgeführt.

Beispiele 1 bis 8 (L-Zeolithe)

Die Reaktionen werden unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch nach üblichen Methoden.

Die in den Beispielen verwendeten Katalysatoren sind:

Katalysator A

Kommerziell erhältlicher L-Zeolith (Baylith L®) wird mit Verformungshilfsmitteln zu 2 mm-Strängen verformt. Nach Trocknen bei 110 °C/16 h und Calcination bei 500 °C/16 h liegt der fertige Katalysator A vor.

Katalysator B (Vergleichskatalysator)

Kommerziell erhältlicher NaY-Zeolith wird mit Boehmit im Gewichtsverhältnis 60 :40 verstrangt, bei 110 °C getrocknet und bei 500 °C/16 h calciniert und mit 20%iger Ammoniumchloridlösung einem Ionenaustausch unterworfen. Der Restnatriumgehalt des Katalysators B beträgt 0,2 Gew.% (500 °C calciniert).

Katalysator C (Vergleichskatalysator)

Boehmit wird mit Verformungshilfsmitteln zu 2 mm Strängen verformt, bei 110 °C/16 h getrocknet und bei 500 °C/16 h calciniert.

Katalysator D (Vergleichskatalysator)

$Al_2O_3$-Stränge (D 10-10®)

Katalysator E (Vergleichskatalysator)

Katalysator E ist ein gefälltes Aluminiumphosphat, das durch Fällung aus $Al(NO_3)_3$-$H_3PO_4$-Lösung mit $NH_3$ bei pH = 6 - 7 erhalten wird. Nach Abfiltrieren des Niederschlags wird bei 110 °C getrocknet und bei 500 °C calciniert. Katalysator E enthält 28,5 Gew.% Al und 13,2 Gew.% P.

In der nachfolgenden Tabelle 1 sind die mit obigen Katalysatoren erzielten Versuchsergebnisse und Versuchsbedingungen aufgezeigt:

Einsatzlösung I: Monohydroxyethylpiperazin in Wasser gelöst 25 g : 75 g.

Einsatzlösung II: Gemisch aus Mono- und Dihydroxyethylpiperazin (Gewichtsverhaltnis 20 : 80) in Wasser gelöst 25 g : 75 g.

Tabelle 1

| Beispiel | 1 | 2* | 3* | 4* | 5 | 6* | 7* | 8* |
|---|---|---|---|---|---|---|---|---|
| Einsatzlösung | I | I | I | I | II | II | II | II |
| Katalysator | A | C | D | E | A | B | D | E |
| Temperatur | 400 °C | 400 °C | 400 °C | 400 °C | 400 °C | 400 °C | 400 °C | 400 °C |
| WHSV | 2 $h^{-1}$ | 2 $h^{-1}$ | 2 $h^{-1}$ | 2 $h^{-1}$ | 2 $h^{-1}$ | 2 $h^{-1}$ | 2 $h^{-1}$ | 2 $h^{-1}$ |
| Umsatz % | 95,7 | 98,2 | 99,9 | 99,9 | 94,8 | 99,6 | 100 | 99,5 |
| Selektivität % | | | | | | | | |
| DABCO | 73,4 | 65,0 | 60,9 | 67,4 | 85,2 | 2,1 | 66,9 | 67,0 |
| Piperazin | 12,2 | 17,1 | 18,2 | 14,6 | 3,0 | 24,0 | 15,3 | 13,3 |

* Vergleichsbeispiel

Beispiele 9 bis 16 (Phosphate mit Zeolithstruktur)

Die Umsetzung wird unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch nach üblichen Methoden.

In den Beispielen werden folgende Katalysatoren verwendet:

Katalysator A

$AlPO_4$-9 (APO-9) wird synthetisiert, indem man 200 g 98 %ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200 °C während 336 h unter autogenem Druck umsetzt. Nach Abfiltrieren wird das kristalline Material bei 120 °C getrocknet und bei 500 °C/16 h calciniert. Das so synthetisierte $AlPO_4$-9 enthält 49,0 Gew.% $P_2O_5$. 37,1 Gew.% $Al_2O_3$. Dieses Material wird mit Verstrangungshilfsmittel zu 3 mm Strängen verformt, abermals bei 120 °C getrocknet und bei 500 °C/6 h calciniert.

Katalysator B

Siliciumaluminiumphosphat-5 (SAPO-5) wird hergestellt aus einer Mischung aus 200 g 98 %ige Phosphorsäure, 136 g Boehmit, 60 g Kieselsol (30 %ig), 287 g Tripropylamin und 587 g $H_2O$. Diese Mischung wird bei 150 °C während 168 h unter autogenem Druck umgesetzt. Nach Filtration wird das kristalline Produkt bei 120 °C getrocknet und bei 500 °C calciniert. SAPO-5 enthält 49,8 Gew.% $P_2O_5$, 33,0 Gew.% $Al_2O_3$, 6,2 Gew.% $SiO_2$. SAPO-5 wird mit einem Verstrangungshilfsmittel zu 3 mm Strängen verformt, bei 120 °C getrocknet und bei 500 °C calciniert.

Katalysator C (Vergleichskatalysator)

Katalysator C ist ein gefälltes Aluminiumphosphat, das durch Fällung aus $Al(NO_3)_3$ und $H_3PO_4$-Lösung mit $NH_3$ bei pH = 6 bis 7 erhalten wird. Nach Abfiltrieren des Niederschlages wird bei 110 °C getrocknet und bei 500 °C calciniert. Katalysator C enthält 28,5 Gew.% Al und 13,2 Gew.% P.

Katalysator D (Vergleichskatalysator)

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3 mm Strängen verformt. Diese Stränge werden bei 100 °C getrocknet und bei 350 °C calciniert. Katalysator D enthält 8,77 Gew.% B und 28,3 Gew.% P.

Katalysator E (Vergleichskatalysator)

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator E enthält 47,1 Gew.% Ce und 12,7 Gew.% P.

Die Ergebnisse und die Versuchsbedingungen sind in Tabelle 1 zusammengestellt.

Einsatzlösung I: Monohydroxyethylpiperazin gelöst in Wasser 25 g : 75 g.

Einsatzlösung II: Gemisch aus 80 Gew.% Di- und 20 Gew.% Monohydroxyethylpiperazin gelöst in Wasser gelöst 25 g : 75 g.

Tabelle 1

| Beispiel | 9 | 10* | 11* | 12* | 13 | 14 | 15* | 16* |
|---|---|---|---|---|---|---|---|---|
| Einsatzlösung | I | I | I | I | II | II | II | II |
| Katalysator | A | C | D | E | A | B | C | D |
| Temperatur | 400 °C | 400 °C | 400 °C | 400 °C | 400 °C | 400 °C | 400 °C | 400 °C |
| WHSV | 2 $h^{-1}$ | 2 $h^{-1}$ | 2 $h^{-1}$ | 2 $h^{-1}$ | 2 $h^{-1}$ | 2 $h^{-1}$ | 2 $h^{-1}$ | 2 $h^{-1}$ |
| Umsatz % | 99,8 | 99,9 | 98,5 | 98,6 | 99,8 | 99,7 | 99,5 | 96,0 |
| Selektivität % | | | | | | | | |
| DABCO | 81,4 | 67,4 | 27,7 | 28,7 | 86,0 | 77,1 | 67,0 | 13,2 |
| Piperazin | 11,1 | 14,6 | 19,8 | 26,4 | 2,7 | 6,5 | 13,3 | 19,7 |

* Vergleichsbeispiel

Beispiele 17 bis 26 (Zirkonphosphate)

Die Reaktion wird unter isothermen Bedingungen in einem Rohrreaktor (Wendel 0,2 cm, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch nach üblichen Methoden.

Die in den Beispielen verwendeten Katalysatoren sind:

Katalysator A

Im Handel erhältliches Zirkonphosphat (CZP 100®) wird mit Verformungshilfsmitteln zu 2 mm-Strängen verformt, bei 110 °C getrocknet und bei 500 °C/16 h calciniert.

Katalysator B (Vergleichskatalysator)

$BPO_4$ wird hergestellt, indem man 49 g $H_3BO_3$ mit 117 g $H_3PO_4$ (75 %ig) in einem Kneter zusammengibt, überschüssiges Wasser abdampft und das Umsetzungsprodukt zu 3 mm-Strängen verformt. Diese Stränge werden bei 100 °C getrocknet und bei 350 °C calciniert. Katalysator B enthält 8,77 Gew.% B und 28,3 Gew.% P.

Katalysator C (Vergleichskatalysator)

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120 °C getrocknet und bei 450 °C calciniert. Katalysator C enthält 47,1 Gew.% Ce und 12,7 Gew.% P.

Katalysator D (Vergleichskatalysator)

Katalysator D ist eine gefälltes Aluminiumphosphat, das durch Fällung aus $Al(NO_3)_3$-$H_3PO_4$-Lösung mit $NH_3$ bis pH = 6-7 erhalten wird. Nach Abfiltrieren des Niederschlags wird bei 110 °C getrocknet und bei 500 °C calciniert. Katalysator D enthält 28,5 Gew.% Al und 13,2 Gew.% P.

Katalysator E (Vergleichskatalysator)

Boehmit wird mit Verformungshilfsmitteln zu 2 mm Strängen verformt, bei 110 °C/16 h getrocknet und bei 500 °C/16 h calciniert.

Katalysator F (Vergleichskatalysator)

$Al_2O_3$-Stränge (D 10-10®)

In der nachfolgenden Tabelle 1 sind die mit obigen Katalysatoren erzielten Versuchsergebnisse und Versuchsbedingungen aufgezeigt:

Einsatzlösung I: Monohydroxyethylpiperazin in Wasser gelöst 25 g : 75 g.

Einsatzlösung II: Gemisch aus Mono- und Dihydroxyethylpiperazin (Gewichtsverhältnis 20 : 80) in Wasser gelöst 25 g : 75 g.

Tabelle 1

| Beispiel | 17 | 18* | 19* | 20* | 21* | 22 | 23* | 24* | 25* | 26* |
|---|---|---|---|---|---|---|---|---|---|---|
| Einsatzlösung | I | I | I | I | I | II | II | II | II | II |
| Katalysator | A | B | C | D | F | A | B | D | E | F |
| Temperatur | 400 °C | 400 °C | 400 °C | 400 °C | 400 °C | 400 °C | 400 °C | 400 °C | 400 °C | 400 °C |
| WHSV | $2h^{-1}$ | $2\ h^{-1}$ | $2\ h^{-1}$ | $2\ h^{-1}$ | $2\ h^{-1}$ | $2\ h^{-1}$ | $2\ h^{-1}$ | $2\ h^{-1}$ | $2\ h^{-1}$ | $2\ h^{-1}$ |
| Umsatz % | 99,8 | 98,5 | 98,6 | 99,9 | 99,9 | 99,7 | 96,0 | 99,5 | 100 | 100 |
| Selektivität % | | | | | | | | | | |
| DABCO | 70,7 | 27,7 | 28,7 | 67,4 | 60,9 | 79,4 | 13,2 | 67,0 | 69,8 | 66,9 |
| Piperazin | 15,1 | 19,8 | 26,4 | 14,6 | 18,2 | 4,7 | 19,7 | 13,3 | 14,7 | 15,3 |

* Vergleichsbeispiel

## Patentansprüche

1. Verfahren zur Herstellung von 1,4-Diazabicyclo(2,2,2)-octan und C-substituierten 1,4-Diazabicyclo-(2,2,2)-octanen der Formel (I)

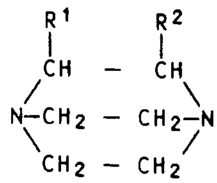

(I),

in der $R^1$ und $R^2$ Wasserstoff-, Alkyl- mit 1 bis 4 C-Atomen oder Alkenylreste mit bis zu 4 C-Atomen bedeuten können, dadurch gekennzeichnet, daß man heterocyclische Amine der Formel (II)

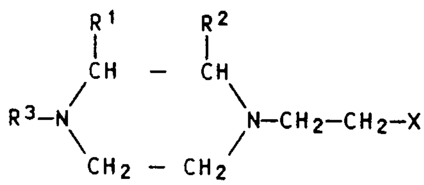

(II),

in der $R^1$ und $R^2$ obige Bedeutung haben und $R^3$ Wasserstoff- oder Hydroxyethyl- oder Aminoethyl- und X Hydroxyl-, Amino-, Hydroxyethyl- oder Aminoethylreste bedeuten, in Gegenwart von L-Zeolithen, Phosphaten mit Zeolithstruktur und Zirkonphosphaten als Katalysatoren umsetzt.

2. Verfahren nach Anspruch 1, dadurch gekennzeichnet, daß man Monohydroxyethylpiperazin und/oder Dihydroxyethylpiperazin als Ausgangsstoffe einsetzt.

3. Verfahren nach Anspruch 1 und 2, dadurch gekennzeichnet, daß man mit Seltenen Erden dotierte L-Zeolithe als Katalysatoren einsetzt.

4. Verfahren nach Anspruch 1 und 2, dadurch gekennzeichnet, daß man mit mit Übergangsmetallen dotierte L-Zeolithe als Katalysatoren einsetzt.

5. Verfahren nach Anspruch 1 und 2, dadurch gekennzeichnet, daß man mit Alkali- und/oder Erdalkalimetallen dotierte L-Zeolithe als Katalysatoren einsetzt.

6. Verfahren nach Anspruch 1 und 2, dadurch gekennzeichnet, daß man mit Phosphorverbindungen dotierte L-Zeolithe als Katalysatoren einsetzt.

7. Verfahren nach Anspruch 1 und 2, dadurch gekennzeichnet, daß man mit Alkalimetallen und Phosphorverbindungen dotierte L-Zeolithe einsetzt.

8. Verfahren nach den Ansprüchen 1 und 2, dadurch gekennzeichnet, daß man hydrothermal hergestellte Phosphate als Katalysatoren verwendet.

9. Verfahren nach Anspruch 8, dadurch gekennzeichnet, daß man mit Alkalimetallen, Erdalkalimetallen, Übergangsmetallen oder Seltenen Erdmetallen dotierte Phosphate als Katalysatoren verwendet.

## Claims

1. A process for the preparation of 1,4-diazabicyclo[2.2.2]octane and C-substituted 1,4-diazabicyclo[2.2.2]-octanes of the formula (I)

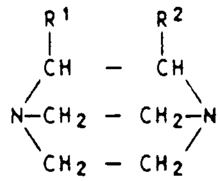

(I)

where $R^1$ and $R^2$ may each be hydrogen, alkyl of from 1 to 4 carbon atoms or alkenyl of not more than 4 carbon atoms, wherein a heterocyclic amine of the formula (II)

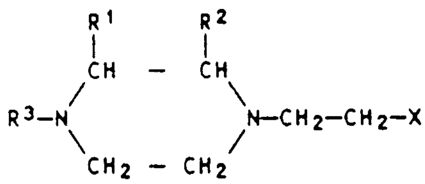

(II)

where $R^1$ and $R^2$ have the above meanings and $R^3$ is hydrogen, hydroxyethyl or aminoethyl and X is hydroxyl, amino, hydroxyethyl or aminoethyl, is reacted in the presence of an L-zeolite, a phosphate having a zeolite structure or a zirconium phosphate as catalyst.

**2.** A process as claimed in claim 1, wherein the starting materials used are monohydroxyethylpiperazine and/or dihydroxyethylpiperazine.

**3.** A process as claimed in claims 1 and 2, wherein the catalysts used are L-zeolites doped with rare earth metals.

**4.** A process as claimed in claims 1 and 2, wherein the catalysts used are L-zeolites doped with transition metals.

**5.** A process as claimed in claims 1 and 2, wherein the catalysts used are L-zeolites doped with alkali metals and/or alkaline earth metals.

**6.** A process as claimed in claims 1 and 2, wherein the catalysts used are L-zeolites doped with phosphorus compounds.

**7.** A process as claimed in claims 1 and 2, wherein L-zeolites doped with alkali metals and phosphorus compounds are used.

**8.** A process as claimed in claims 1 and 2, wherein the catalysts used are hydrothermally prepared phosphates.

**9.** A process as claimed in claim 8, wherein the catalysts used are phosphates doped with alkali metals, alkaline earth metals, transition metals or rare earth metals.

## Revendications

**1.** Procédé de préparation de 1,4-diazabicyclo[2.2.2]octane et de 1,3-diazabicyclo[2.2.2]octanes C-substitués de formule (I)

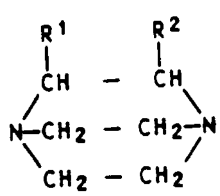

(I),

dans laquelle $R^1$ et $R^2$ peuvent représenter des atomes d'hydrogène, des restes alkyle à 1-4 atomes de carbone ou des restes alcényle renfermant jusqu'à 4 atomes de carbone, caractérisé en ce qu'on fait réagir des amines hétérocycliques de formule (II)

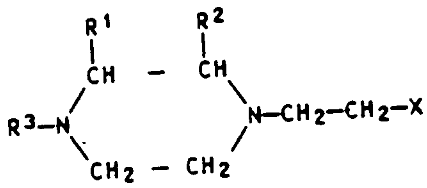

(II),

dans laquelle $R^1$ et $R^2$ ont les significations données ci-dessus, $R^3$ représente un atome d'hydrogène ou un reste hydroxyéthyle ou aminoéthyle et X représente un radical hydroxyle, amino, hydroxyéthyle ou aminoéthyle, en présence de L-zéolithes, de phosphates à structure zéolithique et de phosphates de zirconium en tant que catalyseurs.

2. Procédé selon la revendication 1, caractérisé en ce qu'on utilise, comme substancs de départ, de la monohydroxyéthylpipérazine et/ou de la dihydroxyéthylpipérazine.

3. Procédé selon la revendication 1 ou 2, caractérisé en ce qu'on utilise, comme catalyseurs, des L-zéolithes dopées avec des terres rares.

4. Procédé selon la revendication 1 ou 2, caractérisé en ce qu'on utilise, comme catalyseurs, des L-zéolithes dopées avec des métaux de transition.

5. Procédé selon la revendication 1 ou 2, caractérisé en ce qu'on utilise, comme catalyseurs, des L-zéolithes dopées avec des métaux alcalins et/ou alcalino-terreux.

6. Procédé selon la revendication 1 ou 2, caractérisé en ce qu'on utilise, comme catalyseurs, des L-zéolithes dopées avec des composés du phosphore.

7. Procédé selon la revendication 1 ou 2, caractérisé en ce qu'on utilise, comme catalyseurs, des L-zéolithes dopées avec des métaux alcalins et des composés du phosphore.

8. Procédé selon la revendication 1 ou 2, caractérisé en ce qu'on utilise, comme catalyseurs, des phosphates préparés par voie hydrothermale.

9. Procédé selon la revendication 8, caractérisé en ce qu'on utilise, comme catalyseurs, des phosphates dopés avec des métaux alcalins, des métaux alcalino-terreux, des métaux de transition ou des métaux des terres rares.